(19) Europäisches Patentamt

European Patent Office

Office européen des brevets

(11) **EP 1 378 744 A1**

(12) **EUROPEAN PATENT APPLICATION**

(43) Date of publication:
**07.01.2004 Bulletin 2004/02**

(51) Int Cl.7: **G01N 21/35**, G01N 21/49,
G01N 33/483

(21) Application number: **03253943.9**

(22) Date of filing: **23.06.2003**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **03.07.2002 AU PS332802**

(71) Applicant: **IWRX Pty Ltd.**
**Emerald, Victoria 3782 (AU)**

(72) Inventors:
• **Edye, Leslie, Sugar Research Institute**
**Mackay, QLD 4740 (AU)**
• **Huberts, John of**
**Emerald, Victoria 3782 (AU)**

(74) Representative: **Gillam, Francis Cyril et al**
**SANDERSON & CO.**
**European Patent Attorneys**
**34, East Stockwell Street**
**Colchester Essex CO1 1ST (GB)**

(54) **Sugar monitor**

(57)    An infra red spectrometer incorporates a flow cell connected to a process line containing sugar to provide continuous sugars measurements. The optical components and lamp are selected to operate in the range of 1800nm to 2500nm. The absorption at two wavelengths are used to calculate sucrose concentration. Absorbance at one of the wavelengths is independent of sugars concentration so that the difference between the two is indicative of sugar concentration. The method is applicable to both beet and cane sugars as well as any food processing system which requires monitoring of sugar concentrations.

FIG.4.

## Description

This invention relates to a method and device for determining sugar concentrations in solution as part of a continuous process control.

## Background to the invention

Currently sugar mills do not conduct production line determination of sucrose concentration in processed cane sugar. Off line testing is usually conducted using polarimetry.

USA patent 3609324 discloses a method of determining sucrose concentration by measuring refractive index.

USA 3632446 discloses a method of making invert sugar in which the polarization of the solution is sensed. Polarimetry is also used in USA patent 5317150.

In USA patent 3713738 polarisation and refractive index are measured to obtain a sucrose purity measure.

US patent 6297057 by Matsushita uses angle of rotation to determine urine sugar levels.

It is an object of this invention to provide a method adapted for continuous production line sensing of sugars as well as a device for carrying out such a method.

## Brief description of the invention

To this end the present invention provides a method of continuously measuring the concentration of sugars in solution which includes the steps of

a) passing a portion of the solution being processed through a flow cell
b) irradiating the flow cell with infrared radiation
c) measuring the absorption of radiation of at least one wavelength in the infrared region
d) by reference to a predetermined calibration calculating the concentration of sugars
e) displaying and or storing the calculations.

This invention is partly predicated on the discovery that the concentration of sugars can be linearised against absorption amplitude. The method preferably measures absorption at two wavelengths one of which is independent of sugar concentration and the difference between the two measurements is used to calculate sugars concentration. These wavelengths are selected from within the range 1800 to 2500nm [nanometers]. Absorption at about 2260 to 2320 nm is indicative of sucrose and a second measurement is taken at 2248nm as a reference because the absorbance at this wavelength is independent of sugars concentration because the absorbance intensity of water and sugar cross over at this wavelength. The difference in the two absorbance readings is indicative of sugars concentration. These wavelengths are suitable for cane sugar. For beet sugar which contains betaine with an absorption band near 2248nm a different reference wavelength is chosen. For Betaine containing sucrose two suitable cross over points where intensity is independent of concentration are 2190nm and 2330nm. A wavelength greater than the base line measurement is then chosen to use as the wavelength for measuring the sugar.

Throughout this specification sugars means mono- and di-saccharides and other oligo-sachharides including glucose, fructose, sucrose and lactose. The monosachharides show one I R peak and the disaccharides show a second peak. The instrument of this invention can be used to measure any one of the sugars.

The light source is preferably pulsed to compensate for drift in the electronic components and this can be achieved with a chopper to break a continuous beam or by using a pulsating lamp.

In another aspect the present invention provides a device for measuring sugars concentration which includes

a) a flow cell having an inlet and an out let connectable to a process fluid flow line
b) an infrared source adapted to radiate in the wavelength range of 1800 to 2500 nm located on one side of said flow cell
c) a window on a side of said flow cell opposite said infra red source for receiving radiation
d) pulsing means to create pulses of said infra red radiation
e) an optical detector associated with said window to generate signals indicative of absorbance of the radiation at selected wavelengths
f) a controller adapted to integrate the signals over a predetermined time period and treat the signals to a calibration routine to provide an output indicative of sugar concentration.

The wavelength can be selected from the light source by using any suitable means such as interference filters, tunable filters, diffraction gratings, interferometers etc.

Prior to this invention a production line sugar concentration meter was not available. The device can be used to measure sugar concentration in sugar producing plants but also in any food processing line where sugar concentration needs to be monitored such as in processing fruits or drinks or to monitor lactose levels in dairy based products.

The sugar meter of this invention shows a linear response from 0% to 70% w/v sucrose with a relative standard error of 0.35% at a measurement interval of 30seconds. Temperature changes do not effect the measurement and the instrument is operable in ambient temperatures of up to 80°C.

**Detailed description of the invention**

A preferred embodiment of the invention is illustrated in the drawings in which

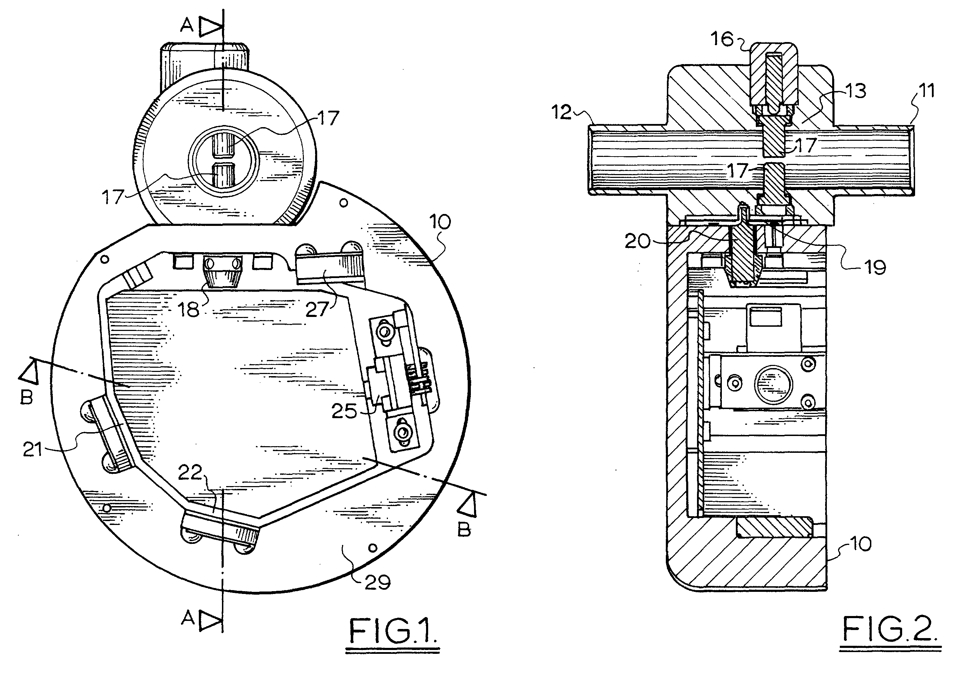

Figure 1 is a side view looking axially along the flow path through the flow cell;

Figure 2 is a view along the line A-A of figure 1;

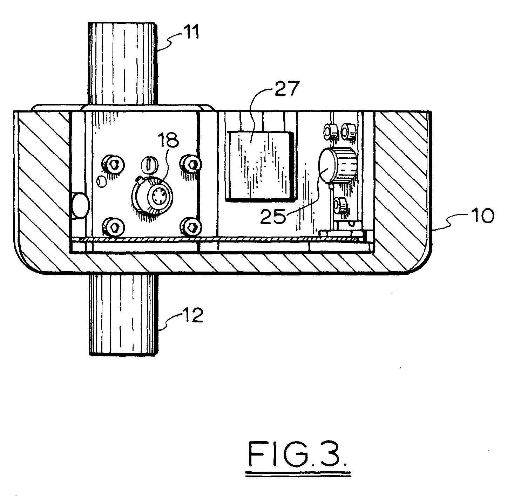

Figure 3 is a view along the line B-B of figure 1;

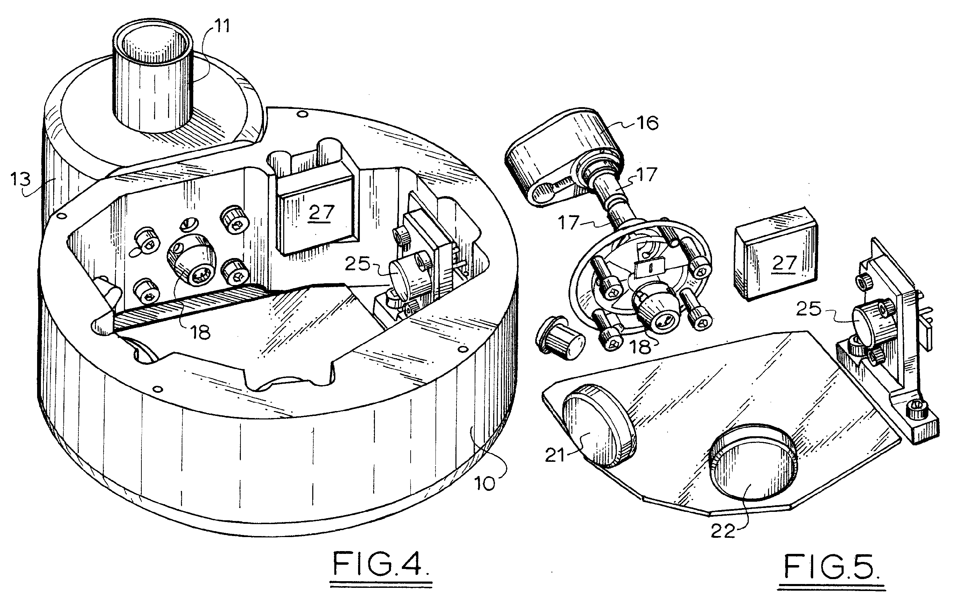

Figure 4 is an isometric view of the device shown in figure 1; and

Figure 5 is a view of the components of figure 4 by removing the housing;

Figure 6 is graph showing absorbance for a 60% sucrose solution compared to the absorbance for water;

Figure 7 is a graph showing the spectra for varying sucrose concentrations in the presence of betaine.

The device consists of a monochromator housing 10 attached to the analysis cell 13 which has an inlet 11 and an outlet 12. Optical windows 17 extend into the cell 13. The electronic controller is contained on a PCB 15. The space 29 may be filled with a dessicant to maintain the optical and electronic components moisture free.

The optical system comprises a source lamp 16, the optical windows 17 a flow through sampling cell 13, an optical spectrometer and an optical detector array 25 all of which are optimised for performance in the 1800 to 2500 nanometre wavelength range.

The optical source 16 is an incandescent tungsten filament lamp but may be a light emitting diode. The light entering the detector system is pulsed and in the embodiment illustrated the pulses are created by breaking the light beam with a chopper 19 driven by an electric motor 20.

The optical spectrometer comprises an entrance slit 18, focusing spherical mirrors 21 and 22 and a diffraction grating 27 arranged in a crossed Czerny-Turner configuration although other configurations may be used. The spectrometer disperses the optical signal according to its wavelength. In this embodiment the diffraction grating 27 is fixed in position and an array detector 25 is used to select the wavelengths of interest for measurement. It is preferred to measure 3 wavelengths.

In other embodiments a single element detector is used and the diffraction grating moved periodically to sequentially select the wavelengths of interest. The bandwidth of the measured optical signals is determined by the width of the entrance slit and the width of the detector element(s).

The optical detector is an array of photoconductive lead sulphide elements although other types of detector may be used.

Sugar Monitor Signal Processing

The optical source lamp is amplitude modulated at a fixed frequency (either electronically or mechanically) and the detector array signals are synchronously demodulated to improve the signal to noise ratio.

The detector array is maintained at a constant temperature below ambient by the use of a thermoelectric cooler, temperature sensing device and control loop to improve the sensitivity and reduce the effect of ambient temperature variations on sensitivity.

The demodulated detector array signals are representative of the intensity of the optical signals at the measured wavelengths. These intensity signals are converted to absorbance signals as follows:

$$A_i = -\log_{10}(I_i)$$

Where $A_i$ is the absorbance of the optical signal at the $i^{th}$ wavelength of the detector array and $I_i$ is the intensity of the optical signal at the $i^{th}$ wavelength of the detector array.

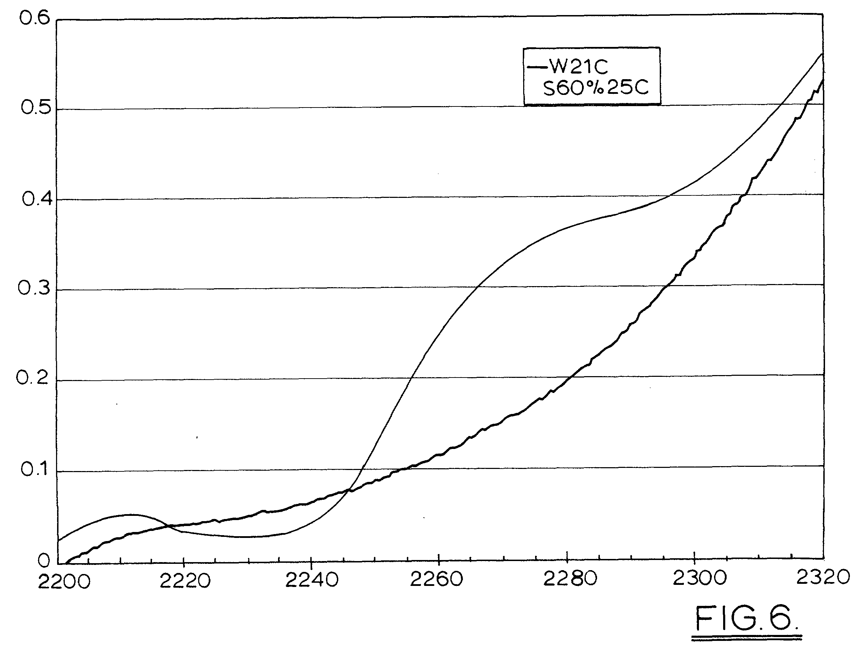

Figure 6 illustrates the observation on which this invention is partly predicated. There is a crossover point in sucrose and water spectra at 2248 nm at which the absorbance is independent of sucrose concentration. For cane sugar the difference in absorbance between 2248nm and 2280 nm is used to calculate sucrose concentration. The absorbance difference has a very high correlation to sucrose concentration (R>0.999).

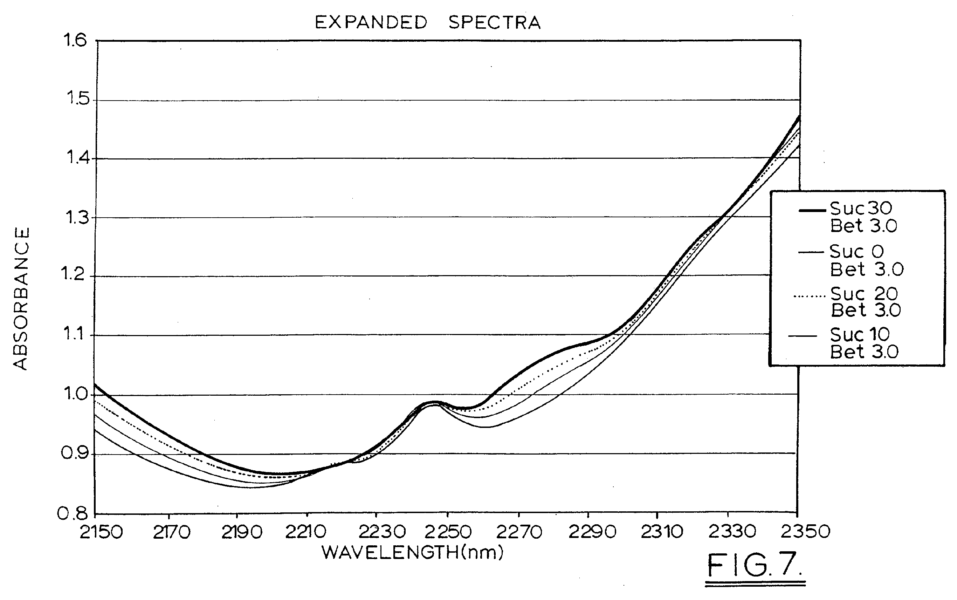

Figure 7 is a similar graph of absorbance when betaine is also present. For betaine containing solutions (from Beet sugar) the baseline measurement are based on a crossover point at 2190nm or 2330nm. For Beet sugar both sucrose and betaine are measured simultaneously using a 3 wavelength detector.

The absorbance signals are integrated over a user specified time period to reduce noise.

The integrated absorbance signals are then applied to a calibration routine to estimate the sucrose concentration. The repeatability of the sucrose measurements in tests is 0.07% w/v.

The sucrose concentration is available for readout to the process control system over a serial communications link.

The response of the instrument to small increases in sugar concentration ( about 0.1% w/v increase between 180 to 200 minutes) indicates that the instrument is of practical use in sugar refining. The continuous monitoring and control of the purity of feeds into vacuum pans has considerable benefit in sugar manufacturing in terms of more consistent sugar quality, better exhaustion of molasses and maximizing throughput of the vacuum pans.

From the above description it can be seen that the present invention provides a unique method and apparatus for production line testing of sucrose concentration. Those skilled in the art will realize that the invention can be adapted to the monitoring of any sugar and can be used as a laboratory instrument even though it is adapted to production line testing in real time.

**Claims**

1. A method of continuously measuring the concentration of a sugars in solution which includes the steps of

   a) passing a portion of the solution being processed through a flow cell
   b) irradiating the flow cell with infrared radiation
   c) measuring the absorption of radiation of at least one wavelength in the infrared region
   d) by reference to a predetermined calibration calculating the concentration of sugars

   displaying and or storing the calculations.

2. A method as claimed in claim 1 in which the infrared radiation is within the range of 1800 to 2500 nm and is pulsed.

3. A method as claimed in claim 1 in which the absorption is derived from infra red detector signals.

4. A method as claimed in claim 1 in which the sugar is sucrose sourced from cane sugar and absorbance is measured at 2248 nm and at a higher wavelength and the difference in absorbance is used as the basis for determining sucrose concentration.

5. A method as claimed in claim 1 in which the sugar is sucrose sourced from beet sugar and absorbance is measured at 2190nm or 2330nm and at a higher wavelength and the difference in absorbance is used as the basis for determining sucrose concentration.

6. A device for measuring sugars concentration which includes

   a) a flow cell having an inlet and an outlet connectable to a process fluid flow line
   b) an infrared source adapted to radiate in the wavelength range of 1800 to 2500 nm located on one side of said flow cell
   c) a window on a side of said flow cell opposite said infra red source for receiving radiation
   d) pulsing means to create pulses of said infra red radiation
   e) an optical detector associated with said window to generate signals indicative of absorbance of the radiation at selected wavelengths

   f) a controller adapted to integrate the signals over a predetermined time period and treat the signals to a calibration routine to provide an output indicative of sugars concentration

7. A device as claimed in claim 6 in which a chopper is used to create the light pulses.

8. A device as claimed in claim 6 in which an array detector is used to select the wavelengths to be measured.

9. A device as claimed in claim 6 in which a diffraction grating is movable to select wavelengths to be detected by a single element detector.

A

17

17

17

10

18

27

25

B

B

21

22

A

29

FIG.1.

16

13

11

12

17

17

20

19

10

FIG.2.

EP 1 378 744 A1

FIG.3.

EP 1 378 744 A1

FIG.5.

FIG.4.

FIG.6.

EP 1 378 744 A1

EXPANDED SPECTRA

FIG.7.

EP 1 378 744 A1

**European Patent Office**

**EUROPEAN SEARCH REPORT**

Application Number

EP 03 25 3943

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | PATENT ABSTRACTS OF JAPAN vol. 2000, no. 19, 5 June 2001 (2001-06-05) -& JP 2001 041884 A (MATSUSHITA ELECTRIC WORKS LTD), 16 February 2001 (2001-02-16) * abstract; figure 1 * | 1-9 | G01N21/35 G01N21/49 G01N33/483 |
| X | PATENT ABSTRACTS OF JAPAN vol. 2002, no. 07, 3 July 2002 (2002-07-03) -& JP 2002 065645 A (MATSUSHITA ELECTRIC WORKS LTD), 5 March 2002 (2002-03-05) * abstract; figure 21 * | 1-9 | |
| X | US 4 655 225 A (DAEHNE CLAUS  ET AL) 7 April 1987 (1987-04-07) * column 3, line 34 - column 4, line 5 * * column 5, line 48 - column 8, line 50; figure 1 * | 1-9 | |
| A | EP 0 317 121 A (KURASHIKI BOSEKI KK) 24 May 1989 (1989-05-24) * abstract; claims 1,5 * | 1-9 | TECHNICAL FIELDS SEARCHED (Int.Cl.7) G01N |
| A | EP 0 282 234 A (DOWLING ELIZABETH MAY) 14 September 1988 (1988-09-14) * column 8, line 1 - line 48 * * column 15, line 33 - column 16, line 6; figure 5 * | 1-9 | |
| A | PATENT ABSTRACTS OF JAPAN vol. 015, no. 421 (C-0878), 25 October 1991 (1991-10-25) & JP 03 173535 A (MATSUSHITA ELECTRIC IND CO LTD), 26 July 1991 (1991-07-26) * abstract * | 1-9 | |

The present search report has been drawn up for all claims

| Place of search | Date of completion of the search | Examiner |
|---|---|---|
| MUNICH | 15 October 2003 | Consalvo, D |

**ANNEX TO THE EUROPEAN SEARCH REPORT
ON EUROPEAN PATENT APPLICATION NO.**

EP 03 25 3943

This annex lists the patent family members relating to the patent documents cited in the above-mentioned European search report.
The members are as contained in the European Patent Office EDP file on
The European Patent Office is in no way liable for these particulars which are merely given for the purpose of information.

15-10-2003

| Patent document cited in search report | | Publication date | Patent family member(s) | | Publication date |
|---|---|---|---|---|---|
| JP 2001041884 | A | 16-02-2001 | NONE | | |
| JP 2002065645 | A | 05-03-2002 | NONE | | |
| US 4655225 | A | 07-04-1987 | NONE | | |
| EP 0317121 | A | 24-05-1989 | JP | 1131436 A | 24-05-1989 |
| | | | JP | 2113713 C | 06-12-1996 |
| | | | JP | 8027235 B | 21-03-1996 |
| | | | AT | 101270 T | 15-02-1994 |
| | | | CA | 1325171 C | 14-12-1993 |
| | | | DE | 3887641 D1 | 17-03-1994 |
| | | | DE | 3887641 T2 | 05-05-1994 |
| | | | EP | 0317121 A2 | 24-05-1989 |
| | | | US | 4883953 A | 28-11-1989 |
| EP 0282234 | A | 14-09-1988 | CN | 1032587 A | 26-04-1989 |
| | | | DK | 111088 A | 18-10-1988 |
| | | | EP | 0282234 A1 | 14-09-1988 |
| | | | FI | 880969 A | 04-09-1988 |
| | | | NO | 880891 A | 05-09-1988 |
| | | | PL | 270977 A1 | 08-12-1988 |
| | | | BR | 8800929 A | 11-10-1988 |
| | | | JP | 63247652 A | 14-10-1988 |
| | | | PT | 86886 A | 30-03-1989 |
| JP 03173535 5 | A | | NONE | | |

EPO FORM P0459

For more details about this annex : see Official Journal of the European Patent Office, No. 12/82